# EUROPEAN PATENT SPECIFICATION

(11) **EP 3 562 520 B1**
(45) Date of publication and mention of the grant of the patent: **10.08.2022**
(21) Application number: 17832701.1
(22) Date of filing: 28.12.2017
(51) Int. Cl.: A61L 15/28, A61L 15/32

(54) **ANTIMICROBIAL WOUND DRESSINGS**
ANTIMIKROBIELLE WUNDVERBÄNDE
PANSEMENTS ANTIMICROBIENS

(30) Priority: 28.12.2016 US 201662439723 P
(43) Date of publication of application: 06.11.2019
(73) Proprietor: Systagenix Wound Management, Limited, Gatwick Airport West Sussex RH3 0PA (GB)
(72) Inventor: WAITE, Alexander, Keighley North Yorkshire BD22 0FN (GB); DELURY, Craig, San Antonio, TX 78265 (US); CULLEN, Breda Mary, Skipton BD23 1HR (GB)
(74) Representative: Simmons & Simmons
(86) International application number: PCT/US2017/068688
(87) International publication number: WO 2018/125993

(56) References cited:
- US-A1- 2006 149 182
- US-A1- 2016 114 074
- FINN GOTTRUP ET AL: "Randomized controlled trial on collagen/oxidized regenerated cellulose/silver treatment : RCT on collagen/ORC/silver in diabetic foot ulcers", WOUND REPAIR AND REGENERATION., vol. 21, no. 2, 1 March 2013 (2013-03-01), pages 216-225, XP055465358, US ISSN: 1067-1927, DOI: 10.1111/wrr.12020
- T. Eberlein ET AL: "Clinical Use of Polihexanide on Acute and Chronic Wounds for Antisepsis and Decontamination", SKIN PHARMACOLOGY AND PHYSIOLOGY: JOURNAL OF PHARMACOLOGICAL ANDBIOPHYSICAL RESEARCH, vol. 23, no. 1, 1 January 2010 (2010-01-01), pages 45-51, XP055735221, CH ISSN: 1660-5527, DOI: 10.1159/000318267

## Description

### CROSS-REFERENCE TO RELATED APPLICATIONS

The present invention claims the benefit, under 35 USC 119(e), of the filing of U.S. Provisional Patent Application serial number 62/439,723, entitled "Antimicrobial Wound Dressings," filed December 28, 2016.

### TECHNICAL FIELD

The present technology relates to compositions and devices, including wound dressings, for application to wounds.

### BACKGROUND

A wide variety of materials and devices, generally characterized as "wound dressings," are known in the art for use in treating an injury or other disruption of tissue, such as wounds. Such wounds may be the result of trauma, surgery, or disease, and affect skin or other tissues. In general, dressings may control bleeding, absorb wound exudate, ease pain, assist in debriding the wound, protect wound tissue from infection, or otherwise promote healing and protect the wound from further damage.

In particular, many wound dressings protect, or assist in the treatment of, infections associated with wounds. Infections can retard wound healing and, if untreated, can result in tissue loss, systemic infections, septic shock and death. A variety of dressings containing antimicrobial agents are known in the art. Nevertheless, there remains a need for improved dressings having one or more characteristics of improved antimicrobial efficacy, improved wound healing, improved absorption of blood and wound exudate, improved wound protection, reduced cost, and greater ease of use. Document US 2006/149182 A1 discloses a wound dressing composition comprising chitosan, ORC and collagen. Silver is present within the composition to form a complex with the ORC.

### BRIEF SUMMARY

The present technology provides wound dressing compositions comprising oxidized regenerated cellulose and poly(hexamethylene biguanide). The compositions preferably also contain a structural protein, such as collagen.

The oxidized regenerated cellulose may be complexed with the poly(hexamethylene biguanide), forming a complex. As referred to herein, such a complex is a mixture of the ORC and PHMB, which may be an intimate mixture at the molecular scale. In some embodiments the complex may comprise ionic or covalent bonding between the ORC and PHMB In some embodiments, the complex may comprise a physical mixture of ORC and PHMB The weight ratio of the ORC to the PHMB is preferably from about 200:1 to about 2000:1.

Preferably, the poly(hexamethylene biguanide) is present in the compositions at a level of from about 0.005% to about 0.02%., more preferably from about 0.008% to about 0.012%.

The compositions may contain an optional gelling agent, such as a polyurethane gel, hydroxyethyl cellulose, hydroxylpropyl cellulose, hydroxypropylmethyl cellulose, modified acrylamide polymer, alginate, pectin, galactomannan, chitosan, hyaluronate, or mixture thereof. The compositions may also contain optional wound healing active materials.

The present technology also provides wound dressings comprising a wound dressing composition. The wound dressing composition may be a component of an absorbent layer in the dressing. The absorbent layer may be in sheet form. The dressing may further comprise a backing sheet having an adhesive margin, and may have an apertured top sheet.

### DRAWINGS

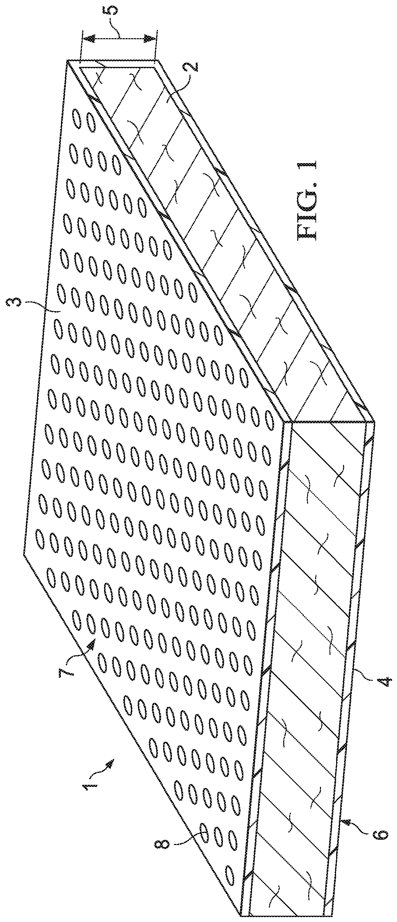

Figure 1 is a perspective view of a wound dressing according to the present technology.

It should be noted that the figure set forth herein is intended to exemplify the general characteristics of materials and methods among those of the present technology, for the purpose of the description of certain embodiments. The figure may not precisely reflect the characteristics of any given embodiment, and is not necessarily intended to define or limit specific embodiments within the scope of this technology.

### DESCRIPTION OF EXAMPLE EMBODIMENTS

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. In particular, the following description sets forth example embodiments and otherwise provides information that enables a person skilled in the art to make and use the subject matter set forth in the appended claims, but may omit certain details already well-known in the art. The following description is, therefore, to be taken as illustrative and not limiting. A non-limiting discussion of terms and phrases intended to aid understanding of the present technology is provided at the end of this Description of Example Embodiments.

The present technology provides wound dressings and compositions useful in wound dressings. Preferably, the materials used in such dressings are physiologically acceptable, commensurate with a reasonable risk/benefit ratio when used in the manner of this technology according to sound medical practice.

The wound dressings of the present technology comprise an absorbent structure, e.g., a "sponge," comprising a wound dressing composition that forms a gel when contacted with an aqueous medium, such as water, blood or wound exudate. In various embodiments, the wound dressing composition is operable to absorb 10 grams, 15 grams, 20 grams, or 25 grams of fluid (e.g, water, blood of wound exudate) per gram of material. In a preferred embodiment, the matrix can absorb 20 grams or less of fluid per gram of material.

### Oxidized Regenerated Cellulose

The wound dressing composition comprises oxidized cellulose, preferably oxidized regenerated cellulose (ORC). Oxidized cellulose may be produced by the oxidation of cellulose, for example with dinitrogen tetroxide. This process converts primary alcohol groups on the saccharide residues to carboxylic acid group, forming uronic acid residues within the cellulose chain. The oxidation may not proceed with complete selectivity, and as a result hydroxyl groups on carbons 2 and 3 may be converted to the keto form. These ketone units introduce an alkali labile link, which at pH 7 or higher initiates the decomposition of the polymer via formation of a lactone and sugar ring cleavage. As a result, oxidized cellulose is biodegradable and bioabsorbable under physiological conditions.

The preferred oxidized cellulose for practical applications is oxidized regenerated cellulose (ORC) prepared by oxidation of a regenerated cellulose, such as rayon. ORC may be manufactured by the process described in U.S. Patent 3,122,479, Smith, issued February 24, 1964. ORC is available with varying degrees of oxidation and hence rates of degradation. In some embodiments, the ORC may be in the form of water-soluble low molecular weight fragments obtained by alkali hydrolysis of ORC.

The ORC may be used in a variety of physical forms, including particles, fibers, sheet, sponge or fabrics. In some embodiments, the ORC is in the form of particles, such as fiber particles or powder particles, for example dispersed in a suitable solid or semisolid topical medicament vehicle. In some embodiments, the wound dressing compositions comprise ORC fibers, wherein a volume fraction of at least 80% of the fibers have lengths in the range of from about 20 µm to about 50 mm. In some embodiments, a volume fraction of at least 80% of the fibers have lengths in the range of from about 5 µm to about 1000 µm, or from about 250 µm to about 450 µm. In some embodiments, a volume fraction of at least 80% of the fibers consists of fibers having lengths in the range of from about 25 mm to about 50 mm. Desired size distributions can be achieved, for example, by milling an ORC cloth, followed by sieving the milled powder to remove fibers outside the range. Fabrics may include woven, non-woven and knitted fabrics.

ORC may be present in the composition at any level appropriate to result in the desired absorbency and rheological characteristics of the wound dressing composition. In general, the ORC may be present in the absorbent structure at a level of from about 10% to about 98% of the absorbent structure. (Unless otherwise indicated, all percentages herein are by weight of the absorbent structure.)

The wound dressing compositions of the present technology comprise a safe and effective amount of poly(hexamethylene biguanide) ("PHMB"), which is also known as polyaminopropyl biguanid ("PAPB") and polyhexanide, having the following general formula. PHMB is a cationic broad spectrum antimicrobial agent. PHMB can be synthesized by a variety of methods, including polycondensation of sodium dicyanamide and hexamethylenediamine. PHMB is commercially available from a variety of sources, including Cosmocil^{®}, commercialized by Lonza Ltd., Basel, Switzerland.

As referred to herein, a "safe and effective" amount of PHMB (or other material used herein) is an amount that is sufficient to have the desired effect (e.g., antimicrobial activity, with respect to PHMB), without undue adverse side effects (such as toxicity, irritation, cell toxicity, or allergic response), commensurate with a reasonable benefit/risk ratio when used in the manner of this technology. The specific safe and effective amount of the PHMB may vary with such factors as the type and quantity of other materials in the composition, the intended use, and the physical condition of the subject on whom the wound dressings are used.

It has been found that compositions of the present technology comprise PHMB at a level of from about 0.005wt% to about 0.02%, more preferably from about 0.008% to about 0.012%, by weight of the composition. In a preferred composition, the PHMB is present at a level of about 0.01%. Without limiting the mechanism, function or utility of present technology, it has been found that such concentrations of PHMB, which are lower than concentrations among those known in the art, provide antimicrobial efficacy while not adversely affective cell viability or proliferation in wound tissue.

### Structural Proteins

As stated above, the wound dressing compositions of the present technology are preferably resorbable sponges, and preferably comprise ORC, PHMB and a structural protein. Thus, in various embodiments, the absorbent structure comprises a structural protein. Exemplary structural proteins may be selected from the group consisting of fibronectin, fibrin, laminin, elastin, collagen, gelatins, and mixtures thereof. Preferably, the structural protein comprises, or is, collagen; in some embodiments, the sponge consists or consists essentially of ORC, PHMB, collagen and, optionally, one or more optional materials as described below.

Collagen useful herein may be obtained from any natural source. The collagen may be Type I, II or III collagen, or may also be chemically modified collagen, for example an atelocollagen obtained by removing the immunogenic telopeptides from natural collagen. The collagen may also comprise solubilised collagen or soluble collagen fragments having molecular weights in the range of from about 5,000 to about 100,000, preferably from about 5,000 to about 50,000, obtained, for example, by pepsin treatment of natural collagen. In various embodiments, the collagen is obtained from bovine corium that has been rendered largely free of non-collagenous components. Such non-collagenous components include fat, non-collagenous proteins, polysaccharides and other carbohydrates, as described in U.S. Patent 4,614,794, Easton et al., issued September 30, 1986 and U.S. Patent 4,320,201, Berg et al., issued March 16, 1982.

The collagen or other structural protein may be present in the wound dressing absorbent structure at a level of from about 1% to about 90% collagen. In various embodiments, the absorbent composition comprises from about 40% to about 70%, or from about 50% to about 60% collagen (by weight of the mixture), e.g., about 55% collagen. In various embodiments, the absorbent structure comprises at least about 85%, or at least about 90%, of the mixture of the ORC and the structural protein.

As discussed above, the wound dressing composition may be resorbable. As referred to herein, a resorbable material or structure is a material which is destroyed, disrupted, disappears, or dissolved upon exposure to physiological fluids (e.g., wound exudate) or processes when used in a method of the present technology, such as when applied to wound tissue. It is understood that such resorption may occur as a result of chemical or physical processes, or both. For example, in various embodiments, the wound dressing composition dissolves in about 8 hours or less, when incubated with simulated wound fluid at a temperature of about 37° C. In various embodiments, the wound dressing composition is bioresorbable such that it is not necessary for the wound dressing to be removed from the tissue to which it is applied during a method of the present technology.

### Optional Materials

The wound dressing compositions may comprise one or more additional optional materials. Such optional components may include, for example, preservatives, stabilizing agents, hydrogels and other gelling agents, plasticizers, matrix strengthening materials, dyestuffs, and actives.

For example, in some embodiments, wound dressing compositions may contain an optional gelling agent, such as a hydrogel. Such gelling agents include those selected from the group consisting of polyurethane gels, modified acrylamide polymers, and hydrophilic polysaccharides. Such hydrophilic polysaccharides useful herein include alginates, chitosan, chitin, guar gums, pectin, starch derivatives, cellulose derivatives (such as hydroxyethyl cellulose, hydroxylpropyl cellulose, and hydroxypropylmethyl cellulose), glycosaminoglycans, galactomannans, chondroitin salts (such as chondroitin sulfate), heparin salts (such as heparin sulfate), hyaluroinic acid and salts thereof, hyaluronates, and mixtures thereof. In various embodiments, the optional gelling agent is an anionic polysaccharide, such as an alginate, hyaluronic acid and salts thereof, and mixtures thereof.

In some embodiments, the wound dressing compositions comprise carboxymethyl cellulose ("CMC"), which modifies the rheological, absorbency, and other structural characteristics of the composition. CMC is derived from cellulose, wherein carboxymethyl groups are bonded to hydroxyl groups in the glucopyranose monomers that make up the cellulose. The CMC may be in salt form, comprising a physiologically acceptable cation, such as sodium (i.e., sodium carboxymethyl cellulose). CMC is commercially available, such as Walocel^{™} (sold by The Dow Chemical Company), Cekol^{®} (sold by CP Kelco). In various embodiments, the matrix provides CMC fibers, as further discussed below. CMC may be present in the composition at any level appropriate to result in the desired absorbency and rheological characteristics of the wound dressing composition. In general, the CMC may be present at a level of from about 50% to about 98% of wound dressing composition. (Unless otherwise indicated, all percentages herein are by weight of the wound dressing composition.)

In some embodiments, the wound dressing compositions contain a strengthening material, which improves the handling characteristics of the wound dressing composition by, for example, decreasing its susceptibility to tearing. A preferred strengthening material comprises non-gelling cellulose fibers. Such "non-gelling" cellulose fibers are substantially water insoluble, produced from cellulose that has not been chemically modified to increase water solubility (as contrasted from carboxymethyl cellulose or other cellulose ethers). Non-gelling cellulose fibers are commercially available, such as Tencel^{®} fibers (sold by Lenzing AG). Such fibers may be processed from a commercially-available continuous length, by cutting into lengths that are, in some embodiments, from about 0.5 to about 5 cm, or from about 2 to about 3 cm in length. The non-gelling cellulose fibers may be present in the composition at any level appropriate to result in the desired physical characteristics of the wound dressing composition. In general, the non-gelling cellulose fibers may be present at a level of from about 10% to about 40% of the wound dressing composition. In some embodiments, the wound dressing compositions comprise the non-gelling cellulose fibers at a level such that the weight ratio of CMC:non-gelling cellulose fibers is from about 6:1 to about 4:1, such as about 5:1.

In some embodiments, the wound dressing compositions of the present technology comprise:
(a) carboxymethyl cellulose, wherein the carboxymethyl cellulose is present in the dressing composition at a level of from about 50% to about 98%;
(b) non-gelling cellulose fibers, wherein the cellulose fibers are present in the dressing composition at a level of from about 10% to about 40%; and
(c) and an ORC/PHMB complex.

The composition may further comprise one or more optional gelling agent(s), such as a hydrogel. Exemplary gelling agents include chitosans, hyaluronates, or mixtures thereof. The composition may further comprise a structural protein, such as collagen.

In various embodiments, the compositions are essentially free of water, wherein no water is added to the composition during its manufacture. However, wound dressing compositions may comprise up to 20% water. Preferably, the compositions contain 10% or less of water.

The wound dressing composition may contain a plasticizer, such as glycerol or other polyhydric alcohol. If present, the plasticizer is present at a level of from about 2% to about 10%.

The wound dressing composition may also comprise one or more active materials which aid in wound healing. Actives include non-steroidal anti-inflammatory drugs, acetaminophen, steroids, optional antibiotics and antiseptics (e.g., silver and chlorhexidine), and growth factors (e.g. fibroblast growth factor or platelet derived growth factor). If present, actives are present in "safe and effective" amounts. Such safe and effective amounts are sufficient to have the desired effect (e.g., antimicrobial activity), without undue adverse side effects (such as toxicity, irritation, or allergic response), commensurate with a reasonable benefit/risk ratio when used in the manner of this technology. The specific safe and effective amount of an active may vary with the active and other factors such as the physical form of the active, the type and quantity of other materials in the composition, the intended use, and the physical condition of the subject on whom the wound dressings are used. In general, such actives are optionally present at a level of from about 0.1% to about 10%.

For example, in various embodiments, the wound dressing composition comprises a growth factor. Growth factors include platelet derived growth factor (PDGF), fibroblast growth factor (FGF), and epidermal growth factor (EGF), and mixtures thereof.

For example, the wound dressing may comprise an optional antimicrobial selected from the group consisting of tetracycline, penicillins, terramycins, erythromycin, bacitracin, neomycin, polymycin B, mupirocin, clindamycin and mixtures thereof. Antiseptics among those useful in the wound dressings include silver, chlorhexidine, povidone iodine, triclosan, sucralfate, quaternary ammonium salts and mixtures thereof. In various embodiments, the wound dressings comprise silver, which may be in metallic form, in ionic form (e.g., a silver salt), or both. For example, the silver may be present in ionic form, such as in a complex with an anionic polysaccharide in the composition. In various embodiments, the wound dressing composition comprises a complex of silver and ORC (a "Silver/ORC Complex"). As referred to herein, such a complex is an intimate mixture at the molecular scale, preferably with ionic or covalent bonding between the silver and the ORC. The Silver/ORC Complex preferably comprises a salt formed between the ORC and Ag ⁺, but it may also comprise silver clusters or colloidal silver metal, for example produced by exposure of the complex to light. The complex of an anionic polysaccharide and silver contained in the materials of the present invention can be made by treating the ORC with a solution of a silver salt. In various embodiments, the silver salt is the salt of silver with a weak acid. Silver/ORC complexes useful herein, and methods of producing such complexes, are described in U.S. Patent 8,461,410, Cullen et al., issued June 11, 2013. Similar processes are described in U.S. Patent 5,134,229, Saferstein et al., issued July 28, 1992. In various embodiments, the Silver/ORC Complex may be present in the wound dressing composition at a level of from about 1% to about 2%. For example, a dressing composition may comprise from about 1% to about 2% of a Silver/ORC Complex (by weight of the composition), wherein the Silver/ORC Complex comprises from about 20% to about 30% (e.g., about 25%) of silver by weight of the ORC.

In other embodiments, however, the wound dressing composition does not contain an optional antimicrobial. That is, the composition consists, or consists essentially of, ORC, PHMB, an optional structural protein, and optional materials other than an antimicrobial.

In some embodiments, such as dressings comprising silver, the wound dressing compositions comprise a dyestuff, which is preferably light-absorbing in the visible region 400-700 nm. Such dyestuffs may be operable to photochemically trap generated free radicals that could otherwise react with the silver in the present compositions, acting as photochemical desensitisers. In various embodiments, the antioxidant dyestuff is selected from the group consisting of aniline dyes, acridine dyes, thionine dyes, bis-naphthalene dyes, thiazine dyes, azo dyes, anthraquinone dyes, and mixtures thereof. For example, the antioxidant dyestuff may be selected from the group consisting of gentian violet, aniline blue, methylene blue, crystal-violet, acriflavine, 9-aminoacridine, acridine yellow, acridine orange, proflavin, quinacrine, brilliant green, trypan blue, trypan red, malachite green, azacrine, methyl violet, methyl orange, methyl yellow, ethyl violet, acid orange, acid yellow, acid blue, acid red, thioflavin, alphazurine, indigo blue, methylene green, and mixtures thereof. If present, the dyestuff may be present at a level of about 0.05% to about 5%, typically about 0.2% to about 2%.

In various embodiments, the wound dressing compositions are freeze dried, such as through lyophilization.

### Wound Dressings

The present technology provides wound dressings comprising one or more wound dressing compositions as described above. In general, with reference to Figure 1, such a wound dressing 1 may comprise an absorbent layer 2, wherein the absorbent layer 2 comprises a wound dressing composition of the present technology. The absorbent layer 2 is preferably in substantially sheet form, i.e., having a generally planar structure with two opposite planar surfaces and a depth (or thickness) 5 orthogonal to the planar surfaces. The wound dressing 1 may have a wound facing surface 7 and an opposite back surface 6. The wound facing surface 7 may have a surface area of from about 1 cm² to about 400 cm². Such "planar" surfaces may have a variety of shapes, including square, rectangular, elliptical, circular or other geometries. It will be understood that the shape and area of the wound facing surface 7 may be customized to the location and type of wound onto which the dressing is to be applied.

In various embodiments, the wound dressings (such as wound dressing 1 in Figure 1) comprise one or more additional layers, also comprising sheet-form compositions. Such additional layers may perform any of a variety of functions in the wound dressings, including adherence of the absorbent layer to the wound or to surrounding tissues, increasing structural rigidity of the wound dressing, protection of the absorbent layer from contact with moisture or other materials in the environment in which the wound dressing is used, protection of a wound surface, eliminating or controlling transport of microbes from the wound (such as from the wound to the absorbent layer), and effecting delivery of actives or other materials to the wound surface. In various embodiments such additional layers are conformable to the wound surface and surrounding tissues, for example, being capable of bending such that the wound-facing surfaces of the wound dressing are in substantial contact with the wound and surrounding tissues. The additional layers may contain a variety of optional materials as described above with respect to the wound dressing composition, including PHMB or other antimicrobials.

In some embodiments, the wound dressing 1 further comprises a backing sheet 4 having a wound-facing surface and an opposite back surface. For example, the back surface of the backing sheet 4 may form at least a portion of the back surface 6 of the wound dressing 1. The backing sheet 4 may support the absorbent layer 2 on the wound-facing surface of the backing sheet, such that the back surface of the absorbent layer 2 is proximate to the wound-facing surface of the backing sheet 4. In some embodiments, the back surface of the absorbent layer 2 is in contact with, preferably adhered to, the wound-facing surface of the backing sheet 4.

Preferably, the backing sheet 4 is substantially liquid-impermeable, although permeable to water vapor. Accordingly, in some embodiments, the backing sheet 4 is not permeable to liquid water or wound exudate. Suitably, the backing sheet 4 may preferably have a moisture vapor transmission rate (MVTR) of the backing sheet 4 alone of 300 to 5000 g/m²/24 hours at 37.5 °C at 100% to 10% relative humidity difference. Preferably, the backing sheet 4 is also microorganism-impermeable.

In various embodiments, polymers for forming the backing sheet 4 include polyurethanes and poly alkoxyalkyl acrylates and methacrylates. In various embodiments, the backing sheet 4 comprises a continuous layer of a high density blocked polyurethane foam that is predominantly closed-cell. Backing sheet materials among those useful herein are disclosed in U.S. Patent 3,645,835, Hodgson, issued February 29, 1972. A suitable backing sheet material is the polyurethane film commercially available as Estane^{®} 5714F (sold by The Lubrizol Corporation).

In various embodiments, the backing sheet 4 thickness is in the range of from about 10 µm to about 100 µm. The surfaces of the backing sheet 4 may have a size and configuration such that an area of the backing sheet 4 extends beyond the absorbent layer 2, i.e., wherein the backing sheet 4 defines a marginal region extending from about 1 mm to about 50 mm, beyond one or more edges of the absorbent layer 2. The absorbent layer 2 may be characterized as an "island" on the backing sheet 4. In various embodiments, the marginal region of the backing sheet 4 (i.e., on the wound-facing surface of the backing sheet) is coated with an adhesive. Thus, when applied to a wound tissue, the marginal area may be used to adhere the wound dressing 1 to tissues surrounding the wound.

Adhesives among those useful here include those known in the art, such as pressure sensitive adhesives. In various embodiments, the adhesive is a pressure sensitive adhesive based on acrylate ester copolymers, polyvinyl ethyl ether, and polyurethane. Pressure sensitive adhesives among those useful herein are disclosed in U.S. Patent 3,645,835, Hodgson, issued February 29, 1972. The basis weight of the adhesive layer may be, for example, from about 20 g/m² to about 250 g/m², or from about 50 g/m² to about 150 g/m².

With further reference to Figure 1, the wound dressing 1 may also comprise a top sheet 3 having a wound-facing surface and a back surface, such that the wound-facing surface of the absorbent layer 2 is proximate to the back surface of the top sheet 3. In some embodiments, the wound-facing surface of the top sheet 3 may form at least a portion of the wound-facing surface 7 of the wound dressing 1. The top sheet 3 is preferably permeable to wound fluids such as blood and wound exudate, allowing such fluids to be absorbed by the absorbent layer 2. In some embodiments (as generally exemplified in Figure 1), the top sheet 3 is perforated, having a pore size that excludes passage of bacteria and other microbes.

In various embodiments, the top sheet comprises a resorbable polysaccharide. For example, polysaccharide material may be selected from the group consisting of alginates, chitosan, chitin, guar gums, starch, starch derivatives, β-Glucans, cellulose, cellulose derivatives, glycosaminoglycans, chondroitin sulfate, heparin sulfate, pectins, and mixtures thereof.

In some embodiments, the resorbable polysaccharide comprises, or is, chitosan. Chitosan is derived from the natural biopolymer, chitin, which is composed of N-acetyl-D-glucosamine units. Chitin may be extracted from the outer shell of shrimps and crabs in known fashion. The chitin is then partially deacetylated, for example by treatment with 5M - 15M NaOH, to produce chitosan. Complete deacetylation of the chitin is not a practical possibility, but preferably the chitosan is at least 50% deacetylated, more preferably at least 75% deacetylated. Chitosan in the free base form is swellable but not substantially soluble in water at near-neutral pH, but soluble in acids due to the presence of ammonium groups on the chitosan chain. The solubility of the chitosan may be reduced by cross-linking, for example with epichlorhydrin. Typically, the average molecular weight of the chitosan as determined by gel permeation chromatography is from about 10⁵ to about 10⁶. Chitosan may be incorporated into the top sheet 3 in any appropriate physical forms, for example, as a film/membrane; sponge; or fiber.

In various embodiments, the top sheet comprises from about 25% to about 75% of the resorbable polysaccharide (e.g., chitosan), by weight of the top sheet. In some embodiments, the solution used to generate the top sheet material has a solids content of from about 1% to about 5%, more preferably from about 1% to about 2%. The top sheet may be made by any of a variety of suitable methods, including casting or molding an aqueous solution comprising the resorbable polysaccharide into a substantially planar sheet structure (as further described below), and drying. The aqueous solution may be generated through combining chitosan with 0.05M Acetic acid at 1.5% w/v with continuous stirring until the solution becomes homogenous. A plasticizer (such as glycerol) may be added so that the resulting film material is flexible. The aqueous solution is then dried at 37°C in a tray for 24 hours to generate a film material.

In various embodiments, the top sheet modulates the exposure of the absorbent structure, such as the absorbent layer 2, to tissue and tissue fluids (for example, tissue exudate comprising bacterial collagenase), so as to control the bioresorption of the of the collagen/ORC sponge. Such modulation may be effected by the chemical composition of the top sheet (e.g., the resorbability of the materials comprising the top sheet) or the physical properties of the top sheet, or both. Accordingly, in various embodiments, the top sheet is resorbable. The rate of resorption may be controlled, however, so as to delay exposure of the absorbent structure to physiological fluids from the wound site or other tissue to which the wound dressing is applied. In some embodiments, the top sheet is intact or exhibits slight degradation when incubated with simulated wound fluid containing enzyme (collagenase at 0.1mg/ml) at a temperature of about 37° C.

In some embodiments, the top sheet 3 comprises a perforation 8, preferably a plurality of perforations. For example, the top sheet 3 may have a perforation density of about 4/cm², and the perforation(s) 8 have an average diameter of from about 0.02 cm to about 0.4 cm.

In some embodiments, a wound dressing comprises a top sheet and bottom sheet, and absorbent structure. Both the top sheet and the bottom sheet have a size and configuration such that an area of each sheet extends beyond the absorbent structure, i.e., wherein the top sheet defines a marginal region extending from about 0.5 mm to about 60 mm, or from about 1 mm to about 50 mm, beyond one or more edges of the absorbent structure, and where the bottom sheet defines a marginal region extending from about 0.5 mm to about 60 mm, or from about 1 mm to about 50 mm, beyond one or more edges of the absorbent structure. The top sheet and bottom sheet may be adhered in the marginal region, e.g., by heat sealing or use of adhesive, to substantially encapsulate the absorbent structure.

Accordingly, in some embodiments, the present technology provides wound dressings comprising:
(a) a bioresorbable sponge having a wound-facing surface and an opposite bottom surface, the structure comprising oxidized regenerated cellulose (ORC), collagen, and PHMB; and
(b) a bioresorbable top sheet covering the wound-facing surface of the bioresorbable sponge, the top sheet comprising a polysaccharide polymer, e.g., chitosan.

Preferably, the top sheet is intact or exhibits slight degradation when incubated with simulated wound fluid containing a collagenase enzyme at a temperature of about 37° C. The top sheet may comprise a plurality of perforations, having a perforation density of about 4/cm², wherein the perforations have an average diameter of from about 0.02 to about 0.4. The wound dressing may further comprise a bottom sheet. The bottom sheet may also comprise a resorbable polymer, preferably chitosan. The top sheet and the bottom sheet may be bonded so as to substantially encapsulate the bioresorbable sponge.

The wound dressings are preferably sterile and packaged in a microorganism-impermeable container.

### Methods of Use

The present technology provides methods of treating a wound, comprising applying to the wound a wound dressing composition, preferably as a component of a wound dressing, as described above. The compositions and dressings may be used with any of a variety of wounds, such as those occurring from trauma, surgery or disease. For example, such wounds may be chronic wounds, venous ulcers, decubitus ulcers or diabetic ulcers. In some embodiments, the wound dressing composition is applied to a wound having a high level of proteases.

### Non-limiting discussion of terminology

The headings (such as "Background" and "Brief Summary") and sub-headings used herein are intended only for general organization of topics within the present disclosure, and are not intended to limit the disclosure of the technology or any aspect thereof. In particular, subject matter disclosed in the "Background" may include novel technology and may not constitute a recitation of prior art. Subject matter disclosed in the "Brief Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition or method.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Components may be also be combined or eliminated in various configurations for purposes of sale, manufacture, assembly, or use. Specific examples are provided for illustrative purposes of how to make and use the compositions and methods of this technology and, unless explicitly stated otherwise, are not intended to be a representation that given embodiments of this technology have, or have not, been made or tested. Equivalent changes, modifications and variations of some embodiments, materials, compositions and methods can be made within the scope of the present technology, with substantially similar results.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features. Moreover, descriptions of various alternatives using terms such as "or" do not require mutual exclusivity unless clearly required by the context, and the indefinite articles "a" or "an" do not limit the subject to a single instance unless clearly required by the context.

As used herein, the words "preferred" or "preferable" refer to embodiments of the technology that afford certain benefits, under certain circumstances. However, other embodiments may also be desirable, under the same or other circumstances. Furthermore, the recitation of one or more desired embodiments does not imply that other embodiments are not useful, and is not intended to exclude other embodiments from the scope of the technology.

Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if parameter X is exemplified herein to have values in the range of 1 - 10, or 2 - 9, or 3 - 8, it is also envisioned that Parameter X may have other ranges of values including 1 - 9, 1 - 8, 1 - 3, 1 - 2, 2 - 10, 2 - 8, 2 - 3, 3 - 10, and 3 - 9.

Although the open-ended term "comprising," as a synonym of non-restrictive terms such as including, containing, or having, is used herein to describe and claim embodiments of the present technology, embodiments may alternatively be described using more limiting terms such as "consisting of' or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

The example embodiments may also be described herein with reference to spatial relationships between various elements or to the spatial orientation of various elements depicted in the attached drawings. For example, such relationships or orientations as "top" or "bottom" assume a frame of reference consistent with an exemplary special orientation of a wound dressing. However, as would be recognized by those skilled in the art, this frame of reference is merely a descriptive expedient rather than a strict prescription as to the orientation of any given dressing as manufactured or used.

The appended claims set forth novel and inventive aspects of the subject matter described above. Features, elements, and aspects described herein may also be combined or replaced by alternative features serving the same, equivalent, or similar purpose without departing from the scope of the invention defined by the appended claims.

## Claims

1. A wound dressing composition comprising:
(a) oxidized regenerated cellulose (ORC); and
(b) poly(hexamyethylene biguanide) (PHMB), at a level of from about 0.005wt% to about 0.02wt% by weight of the composition, preferably from about 0.008wt% to about 0.012wt% by weight of the composition.

2. The wound dressing composition according to Claim 1, comprising a complex of the ORC and the PHMB.

3. The wound dressing composition according to Claim 1 or 2, wherein the weight ratio of the ORC to the PHMB is from about 200:1 to about 2000:1.

4. The wound dressing composition according to any of the preceding claims, further comprising a structural protein.

5. The wound dressing composition according to Claim 4, wherein the structural protein is:
selected from the group consisting of fibronectin, fibrin, laminin, elastin, collagen, gelatins, and mixtures thereof; or
comprises collagen.

6. The wound dressing composition according to any one of the preceding claims, further comprising a polysaccharide gelling agent, wherein the polysaccharide gelling agent is selected from the group consisting of alginates, chitosan, chitin, guar gums, pectin, starch, cellulose, glycosaminoglycans, galactomannans, chondroitin salts, heparin salts, hyaluronic acid and salts thereof, and mixtures thereof, and optionally wherein the polysaccharide gelling agent comprises chitosan.

7. The wound dressing composition according to any one of claims 1-5, further comprising a gelling agent selected from the group consisting of polyurethane gels, cellulose ethers (e.g., hydroxyethyl cellulose, hydroxyl propyl cellulose, and hydroxypropylmethyl cellulose), modified acrylamide polymers, and mixtures thereof.

8. The wound dressing composition according to any one of the preceding claims, further comprising a wound healing agent, wherein the wound healing agent is selected from the group consisting of non-steroidal anti-inflammatory drugs, steroids, anti-inflammatory cytokines, anaesthetics, antimicrobial agents, growth factors, and mixtures thereof, and optionally further comprising a growth factor selected from the group consisting of platelet derived growth factor (PDGF), fibroblast growth factor (FGF), and epidermal growth factor (EGF), preferably PDGF.

9. The wound dressing composition according to any of the preceding claims, further comprising:
(i) carboxymethyl cellulose, wherein the carboxymethyl cellulose is present in the wound dressing composition at a level of from about 50% to about 98%; and
(ii) non-gelling cellulose fibers, wherein the cellulose fibers are present in the wound dressing composition at a level of from about 10% to about 40%.

10. The wound dressing composition according to any of the preceding claims, wherein the composition is lyophilized.

11. A wound dressing comprising an absorbent layer comprising a wound dressing composition according to any one of the preceding claims.

12. The wound dressing according to Claim 11, wherein the absorbent layer is in sheet form having a wound facing surface and an opposite back surface, and wherein the wound facing surface has a surface area of from about 1 cm² to about 400 cm².

13. The wound dressing according to Claim 11 or Claim 12, further comprising a backing sheet having a wound-facing surface, wherein the wound-facing surface of the backing sheet substantially covers the back surface of the absorbent layer.

14. The wound dressing according to Claim 13, wherein the backing sheet has a surface area larger than the surface area of the back surface of the absorbent layer, the backing sheet having a marginal region extending from about 1 mm to 50 mm beyond two or more edges of the absorbent layer, optionally wherein the wound-facing surface of the backing sheet is coated with an adhesive in the marginal region.

15. The wound dressing according to any one of Claims 11-14, further comprising an apertured top sheet substantially covering the wound facing surface of the absorbent layer.

## Patentansprüche

1. Eine Wundverbandzusammensetzung, umfassend:
(a) oxidierte regenerierte Cellulose (ORC); und
(b) Poly(hexamyethylenbiguanid) (PHMB), in einer Höhe von zu etwa 0,005 Gew.-% bis etwa 0,02 Gew.-% der Zusammensetzung, vorzugsweise von zu etwa 0,008 Gew.-% bis etwa 0,012 Gew.-% der Zusammensetzung.

2. Die Wundverbandzusammensetzung nach Anspruch 1, umfassend einen Komplex der ORC und des PHMB.

3. Die Wundverbandzusammensetzung nach Anspruch 1 oder 2, wobei das Gewichtsverhältnis der ORC zu dem PHMB von etwa 200 : 1 bis etwa 2000 : 1 beträgt.

4. Die Wundverbandzusammensetzung nach einem der vorstehenden Ansprüche, ferner umfassend ein Strukturprotein.

5. Die Wundverbandzusammensetzung nach Anspruch 4, wobei das Strukturprotein:
ausgewählt ist aus der Gruppe bestehend aus Fibronektin, Fibrin, Laminin, Elastin, Kollagen, Gelatinen und Mischungen davon; oder
Kollagen umfasst.

6. Die Wundverbandzusammensetzung nach einem der vorstehenden Ansprüche, ferner umfassend ein Polysaccharid-Geliermittel, wobei das Polysaccharid-Geliermittel ausgewählt ist aus der Gruppe bestehend aus Alginaten, Chitosan, Chitin, Guargummis, Pektin, Stärke, Cellulose, Glykosaminoglykanen, Galactomannanen, Chondroitinsalzen, Heparinsalzen, Hyaluronsäure und Salzen davon und Mischungen davon, und wobei wahlweise das Polysaccharid-Geliermittel Chitosan umfasst.

7. Die Wundverbandzusammensetzung nach einem der Ansprüche 1 bis 5, ferner umfassend ein Geliermittel, ausgewählt aus der Gruppe bestehend aus Polyurethangelen, Celluloseethern (z. B. Hydroxyethylcellulose, Hydroxylpropylcellulose und Hydroxypropylmethylcellulose), modifizierten Acrylamidpolymeren und Mischungen davon.

8. Die Wundverbandzusammensetzung nach einem der vorstehenden Ansprüche, ferner umfassend ein Wundheilungsmittel, wobei das Wundheilungsmittel ausgewählt ist aus der Gruppe bestehend aus nichtsteroidalen entzündungshemmenden Arzneimitteln, Steroiden, entzündungshemmenden Zytokinen, Anästhetika, antimikrobiellen Mitteln, Wachstumsfaktoren und Mischungen davon, und wahlweise ferner umfassend einen Wachstumsfaktor, ausgewählt aus der Gruppe bestehend aus von Blutplättchen abgeleitetem Wachstumsfaktor (PDGF), Fibroblasten-Wachstumsfaktor (FGF) und epidermalem Wachstumsfaktor (EGF), vorzugsweise PDGF.

9. Die Wundverbandzusammensetzung nach einem der vorstehenden Ansprüche, ferner umfassend:
(i) Carboxymethylcellulose, wobei die Carboxymethylcellulose in der Wundverbandzusammensetzung in einer Höhe von etwa 50 % bis etwa 98 % vorhanden ist; und
(ii) nichtgelierende Cellulosefasern, wobei die Cellulosefasern in der Wundverbandzusammensetzung in einer Höhe von etwa 10 % bis etwa 40 % vorhanden sind.

10. Die Wundverbandzusammensetzung nach einem der vorstehenden Ansprüche, wobei die Zusammensetzung lyophilisiert ist.

11. Der Wundverband, umfassend eine Absorptionsschicht, die eine Wundverbandzusammensetzung nach einem der vorstehenden Ansprüche umfasst.

12. Der Wundverband nach Anspruch 11, wobei die Absorptionsschicht in Lagenform, die eine wundseitige Oberfläche und eine gegenüberliegende rückseitige Oberfläche aufweist, vorliegt, und wobei die wundseitige Oberfläche eine Oberfläche von etwa 1 cm² bis etwa 400 cm² aufweist.

13. Der Wundverband nach Anspruch 11 oder Anspruch 12, ferner umfassend eine Unterlage, die eine wundseitige Oberfläche aufweist, wobei die wundseitige Oberfläche der Unterlage im Wesentlichen die rückseitige Oberfläche der Absorptionsschicht bedeckt.

14. Der Wundverband nach Anspruch 13, wobei die Unterlage eine Oberfläche aufweist, die größer ist als die Oberfläche der rückseitigen Oberfläche der Absorptionsschicht, wobei die Unterlage einen Randbereich aufweist, der sich von etwa 1 mm bis 50 mm über zwei oder mehrere Kanten der Absorptionsschicht hinaus erstreckt, wobei wahlweise die wundseitige Oberfläche der Unterlage mit einem Klebstoff in dem Randbereich beschichtet ist.

15. Der Wundverband nach einem der Ansprüche 11 bis 14, ferner umfassend eine mit Öffnungen versehene Oberlage, die im Wesentlichen die wundseitige Oberfläche der Absorptionsschicht bedeckt.

## Revendications

1. Composition pour pansement pour plaie comprenant :
(a) de la cellulose régénérée oxydée (ORC) ; et
(b) du poly(hexaméthylène biguanide) (PHMB), à un taux allant d'environ 0,005 % en poids à environ 0,02 % en poids de la composition, de préférence allant d'environ 0,008 % en poids à environ 0,012 % en poids de la composition.

2. Composition pour pansement pour plaie selon la revendication 1, comprenant un complexe de l'ORC et du PHMB.

3. Composition pour pansement pour plaie selon la revendication 1 ou 2, dans laquelle le rapport pondéral de l'ORC sur le PHMB va d'environ 200:1 à environ 2 000:1.

4. Composition pour pansement pour plaie selon l'une quelconque des revendications précédentes, comprenant en outre une protéine structurale.

5. Composition pour pansement pour plaie selon la revendication 4, dans laquelle la protéine structurale est :
choisie dans le groupe constitué par la fibronectine, la fibrine, la laminine, l'élastine, le collagène, les gélatines, et les mélanges de ceux-ci ; ou
comprend du collagène.

6. Composition pour pansement pour plaie selon l'une quelconque des revendications précédentes, comprenant en outre un agent gélifiant polysaccharidique, dans laquelle l'agent gélifiant polysaccharidique est choisi dans le groupe constitué par les alginates, le chitosane, la chitine, les gommes de guar, la pectine, l'amidon, la cellulose, les glycosaminoglycanes, les galactomannanes, les sels de chondroïtine, les sels d'héparine, l'acide hyaluronique et les sels de celui-ci, et les mélanges de ceux-ci, et éventuellement dans laquelle l'agent gélifiant polysaccharidique comprend du chitosane.

7. Composition pour pansement pour plaie selon l'une quelconque des revendications 1 à 5, comprenant en outre un agent gélifiant choisi dans le groupe constitué par les gels de polyuréthane, les éthers de cellulose (par exemple, l'hydroxyéthylcellulose, l'hydroxypropylcellulose, et l'hydroxypropylméthylcellulose), les polymères d'acrylamide modifiés, et les mélanges de ceux-ci.

8. Composition pour pansement pour plaie selon l'une quelconque des revendications précédentes, comprenant en outre un agent de cicatrisation de plaie, dans laquelle l'agent de cicatrisation de plaie est choisi dans le groupe constitué par les médicaments anti-inflammatoires non stéroïdiens, les stéroïdes, les cytokines anti-inflammatoires, les anesthésiques, les agents antimicrobiens, les facteurs de croissance, et les mélanges de ceux-ci, et comprenant en outre éventuellement un facteur de croissance choisi dans le groupe constitué par le facteur de croissance dérivé des plaquettes (PDGF), le facteur de croissance des fibroblastes (FGF), et le facteur de croissance épidermique (EGF), de préférence le PDGF.

9. Composition pour pansement pour plaie selon l'une quelconque des revendications précédentes, comprenant en outre :
(i) de la carboxyméthylcellulose, dans laquelle la carboxyméthylcellulose est présente dans la composition pour pansement pour plaie à un taux allant d'environ 50 % à environ 98 % ; et
(ii) des fibres de cellulose non gélifiantes, dans laquelle les fibres de cellulose sont présentes dans la composition pour pansement pour plaie à un taux allant d'environ 10 % à environ 40 %.

10. Composition pour pansement pour plaie selon l'une quelconque des revendications précédentes, dans laquelle la composition est lyophilisée.

11. Pansement pour plaie comprenant une couche absorbante comprenant une composition pour pansement pour plaie selon l'une quelconque des revendications précédentes.

12. Pansement pour plaie selon la revendication 11, dans lequel la couche absorbante est sous forme de feuille ayant une surface faisant face vers la plaie et une surface arrière opposée, et dans lequel la surface faisant face vers la plaie a une aire de surface allant d'environ 1 cm² à environ 400 cm².

13. Pansement pour plaie selon la revendication 11 ou la revendication 12, comprenant en outre une feuille de support ayant une surface faisant face vers la plaie, dans lequel la surface faisant face vers la plaie de la feuille de support recouvre essentiellement la surface arrière de la couche absorbante.

14. Pansement pour plaie selon la revendication 13, dans lequel la feuille de support a une aire de surface supérieure à l'aire de surface de la surface arrière de la couche absorbante, la feuille de support ayant une région marginale s'étendant d'environ 1 mm à 50 mm au-delà de deux bords ou plus de la couche absorbante, éventuellement dans lequel la surface faisant face vers la plaie de la feuille de support est revêtue d'un adhésif dans la région marginale.

15. Pansement pour plaie selon l'une quelconque des revendications 11 à 14, comprenant en outre une feuille supérieure perforée recouvrant essentiellement la surface faisant face vers la plaie de la couche absorbante.
